# EUROPEAN PATENT APPLICATION

(11) **EP 0 990 432 A1**
(43) Date of publication of application: **05.04.2000**
(21) Application number: 99304392.6
(22) Date of filing: 04.06.1999
(51) Int. Cl.: A61F 13/12, A45D 44/12

(54) **An adhesive strip**

(30) Priority: 04.10.1998 IL 12644698
(71) Applicant: Hatam, Ben, Neve Yakov, Jerusalem (IL); Dahuk, Shlomo, Mevaserret Tzion, Jerusalem (IL)
(72) Inventor: Hatam, Ben, Neve Yakov, Jerusalem (IL); Dahuk, Shlomo, Mevaserret Tzion, Jerusalem (IL)
(74) Representative: Ablewhite, Alan James

(57) **Abstract**

The invention provides an adhesive strip for preventing fluid communication between hair treatment agents and a user's skin, comprising a barrier strip having a thickness of about 1-8 mm and a width of about 4-10 mm, adapted to have a bottom surface thereof attached adjacent the hairline of a user, a layer of heat-activated adhesive provided on the bottom surface of the barrier strip, the adhesive being self-sealing at body temperatures, and a removable release layer attached to the adhesive, a longitudinal edge of the adhesive coated bottom surface being adapted for contact with the hairline, and a major portion of the adhesive coated bottom surface of the strip being adapted to conform to a spatial curve defined by the contours of the hairline without crimping or buckling, the strip and adhesive forming a continuous leak-proof barrier adjacent the user's hairline after attachment to the user's skin.

## Description

### Field of the Invention

The present invention is directed to an adhesive strip for preventing fluid communication between hair treatment agents and a user's skin and a method of utilizing the same.

### Background of the Invention

The use of numerous treatment agents on one's body is wide spread around the world. Various medical, cosmetic and hair treatments require the application of treatment agents to specific bodily parts/locations. The manufacturers of said agents usually advise the users of the effects incurred by the contact of the treatment agents with bodily parts other than the treated ones. The above effects range from irritation to the skin to irreversible medical damage, such as the loss of eye-sight.

One optional effect may occur when a treatment agent such as hair dye contacts a user's forehead, causing skin-staining which may remain visible for extended periods of time. Presently, a process such as hair dyeing may last over an hour, during which constant observations for leakage of the hair dye is necessary for eliminating the staining of adjacent skin areas. Furthermore, if fluid communication is permitted between a user's hair and face the dye may come into contact with one's eye causing severe damage. The unwanted contact between hair dye and adjacent skin requires the applier of said dye (whether a hair stylist or the users themselves) to be on constant alert for leakage. Such attention is time consuming and limits the amount of simultaneous customers per practitioner (i.e. hair stylist).

U.S. Patent 5,591,447 discloses an improved wound dressing having an adhesive layer containing one or more hydrocolloids dispersed in a viscous elastomer, a flexible water-impervious backing layer on one side of the adhesive layer, and a removable release layer on the adhesive layer's opposite side. The dressing includes a body portion with a stair-like outer contour merging with a peripheral flange of reduced thickness. The flange is required to have a width of at least 5 mm and the thickness of the adhesive layer of that flange is no greater than 0.5 mm, for the purposes of overcoming prior problems of adhesive flow, fluid channeling, leakage and unintentional detachment.

U.S. Patent no. 5,658,270 provides absorbent sanitary protection products for direct attachment to the skin and hair of a user. The products comprise an absorbent core, a barrier layer coupled on one side of the core and a pressure sensitive adhesive layer on the side of the core that faces the body of a user. The pressure sensitive adhesive has sufficient adhesive strength to enable the product to adhere securely to the skin and hair of the user and yet to be removed without pain or trauma. Products described include those that are restricted to covering and adhering to the perineal area as well as those having ancillary structures to attach to body structures beyond the perineal area.

As will be noted the above patents may serve a different purpose than that of preventing the leakage of hair treatment agents to adjacent skin. An adhesive strip for preventing such leakage should have the following prominent features: flexibility of application to complement the contours of a user's hair line, low adhesiveness at first to allow readjustment of the strip's position, higher adhesiveness after readjustment period, resistance to high-temperatures such as those applied by a hair dryer, self-sealing properties and resistance to the compounds within the treatment agent.

All of the above features may contribute to an adhesive strip forming a continuous leak-proof barrier adjacent the user's hairline after attachment to the user's skin.

The present inventor has unsuccessfully tested the following materials and methods before arriving at the present invention as defined herein:

Closed cell foams such as polyethylene, ethylene vinyl acetate (EVA), polyvinyl chloride (PVC), ethylene propylene diene terpolymer (EPDM), or polychloroprene (neoprene) and silicone foams were not sufficient because the strip tended to straighten and defy bending. This caused more wrinkles in the skin, under which the treating liquid leaked and channeled.

Various types of putty which could form a physical barrier between the hair and adjacent skin were tested. This approach encountered the following problems:

The putty is sticky in all directions and may stick to the hair, preventing the hair dye or other treating agents from contacting the hair.

The stickiness of the putty makes it hard to apply, since it sticks to the hands and fingers. When removing said putty from the skin, the putty leaves residues on the skin. Decreasing the adhesion of such a putty results in a putty which does not stick well enough to the skin.

Elastic material, such as rubber, which can bend and follow the hairline, in combination with an adhesive was not good, since the stretching of rubber during the application to the skin resulted in wrinkles in the skin when the rubber returned to its original position.

The method of dispensing a self-curing polymeric coating from a tube could not be easily carried out by an individual treating his/her own skin or hair. Furthermore, the volume of material needed to form a physical barrier (4-8 mm thick, 5-10 mm wide) around the hairline or around large areas of skin is large.

A readily available adhesive-backed foam, such as weather proof window sealing strips were tested. These are usually closed cell strips suitable for proofing windows, but cannot bend closely enough to complement the contours of a hairline.

In order to overcome the short-comings of prior products the present invention now provides an adhesive strip for preventing fluid communication between hair treatment agents and a user's skin, comprising:
a) a barrier strip having a thickness of about 1-8 mm and a width of about 4-10 mm, adapted to have a bottom surface thereof attached adjacent the hairline of a user;
b) a layer of heat-activated adhesive provided on the bottom surface of said barrier strip, said adhesive being self-sealing at body temperatures; and
c) a removable release layer attached to said adhesive,
a longitudinal edge of said adhesive coated bottom surface being adapted for contact with said hairline, and a major portion of the adhesive coated bottom surface of said strip being adapted to conform to a spatial curve defined by the contours of the hairline without crimping or buckling, said strip and adhesive forming a continuous leak-proof barrier adjacent the user's hairline after attachment to the user's skin,.

In a preferred embodiment of the present invention said barrier strip is made of an open cell material selected from the group consisting of polyurethane, silicone, natural rubber, styrene butadiene rubber (SBR), polychloroprene, nitrile rubber, styrene butadiene styrene (SBS) rubber, styrene isoprene styrene (SIS) and polyvinyl chloride (PVC), wherein said open cell material has a pore size of about 0.3 to 0.6 mm.

In another preferred embodiment said barrier strip is made of a non-woven fabric wherein said non-woven fabric is selected from the group consisting of viscose, polyester, polypropylene, polyethylene, paper and crepe paper.

In a further embodiment said barrier strip is felt.

In an even further embodiment said adhesive is a pressure-sensitive adhesive wherein said adhesive exhibits increased adhesive properties as temperature approaches body temperature and decreased adhesive properties as a function of time due to increased amounts of body secretions contacting said adhesive, wherein said adhesive comprises about 20% to 85% wt/wt of a tackifier, wherein said tackifier is selected from the group consisting of hydrocarbon resin, phenolic resin, polyterpene resin, terpene phenolic resin, wood rosin, hydrogenated wood rosin and colophonium, wherein said adhesive comprises about 10%-35% wt/wt polymer base, wherein said polymer base is a block co-polymer comprising at least one component which is compatible with body fat, wherein said component is selected from the group consisting of styrene butadiene styrene block polymer, styrene isoprene styrene block polymer, natural rubber, butyl rubber and styrene butadiene rubber, wherein said adhesive comprises about 5% to 70% wt/wt plasticizer which is compatible with body fat, wherein said plasticizer is selected from the group consisting of parafinic oils, naphthinic oils, aromatic oils, parafin wax, dibutyl phthalate, dioctyl phthalate and polyolefinic oil, wherein said polyolefinic oil is selected from the group consisting of polybutene and polyisobutene.

The present invention also provides a method for preventing fluid communication between hair treatment agents and a user's skin, comprising:
i) providing an adhesive strip, said strip comprising:
   a) a barrier strip having a thickness of about 1-8 mm and a width of about 4-10 mm, adapted to have a bottom surface thereof attached adjacent the hairline of a user, said strip being made of a material characterized in that a longitudinal edge of said adhesive coated bottom surface is adapted for contact with said hairline, and a major portion of the adhesive coated bottom surface of said strip is adapted to conform to a spatial curve defined by the contours of the hairline without crimping or buckling;
   b) an adhesive provided on the bottom surface of said strip; and
   c) a removable release layer attached to said adhesive,
ii) removing said release layer; and
iii) applying said adhesive strip along the hairline of the user,
whereby said strip and adhesive forms a continuous leak-proof barrier adjacent thereto.

The adhesive strip of the present invention is a flexible strip of significant thickness, which can serve as a physical barrier against spillage and leakage of the treating agents.

The strip is very flexible and it is able to closely follow the contours of the hair line or body and face lines.

The strip is affixed to the body by a self-sealing adhesive having special characteristics.The adhesive layer is quite thick (0.1 mm) and its characteristics enable the adhesive strip to adhere to the skin, including entering the crevices and wrinkles in the skin. This is essential in order to prevent fluid channeling, leakage and unintentional detachment. The initial tack of the adhesive strip is relatively low. This feature allows the user to correct the position of said strip over the skin, by quickly removing it and re-affixing in a different position if needed. As the temperature rises (because of the higher body temperature), the adhesion strengthens and said strip is secured to the skin.

The composition of the adhesive is such that it is not affected by skin oils. Nevertheless, the skin oils allow easy removal of the adhesive strip from the skin without pain or trauma. Even adhesion to the hair will not usually cause hair removal.

A release paper or polymer usually protects the sticky side of the strip until application. Being very flexible, the adhesive strip can be closely wound into a dispenser and dispensed to the skin or hairline as needed.

The pressure sensitive adhesive to be used in such a device has to meet several contradicting needs and requirements:
a) It should be easily affixed to oily, rough skin;
b) it should be easily affixed to skin with varying pH levels;
c) it should be initially easy to remove from the skin, while being strongly adhesive after some time. This need is especially crucial when an individual is using the device on his/her own body without help; and
d) while being strongly secured to the skin during the course of treatment, it should be easily removed from the skin at the end of the treatment without causing pain or inconvenience.

While the invention will now be described in connection with certain preferred embodiments in the following example and with reference to the accompanying figures so that aspects thereof may be more fully understood and appreciated, it is not intended to limit the invention to these particular embodiments. On the contrary, it is intended to cover all alternatives, modifications and equivalents as may be included within the scope of the invention as defined by the appended claims. Thus, the following examples which include preferred embodiments will serve to illustrate the practice of this invention, it being understood that the particulars shown are by way of example and for purposes of illustrative discussion of preferred embodiments of the present invention only and are presented in the cause of providing what is believed to be the most useful and readily understood description of formulation procedures as well as of the principles and conceptual aspects of the invention.

### Example

An adhesive composition is prepared from the following:

| | |
|---|---|
| Hydrogenated wood rosin | 59% |
| Styrene isobutene styrene SIS | 33% |
| Polybutene | 7% |
| *Stabilizer - anti oxidant | 1% |

- The stabilizer does not add to the properties of the adhesive and is added to prevent oxidation. The anti-oxidant used is Irganox 10-10 by Ciba, the chemical composition of which is penta erytrityl tetrakis (3-(3,5-di-tert-butyl-
- 4-hydroxyphenyl)-proprionate)

Said adhesive is attached to the bottom side of a strip of open-cell polyurethane having a thickness 5 mm and a width of 7 mm.

In the drawings:
FIG. 1 is a schematic view of an adhesive strip having a barrier strip made of an open cell material according to the present invention;
FIG. 2 is a schematic view of an adhesive strip having a barrier strip made of felt;
FIG. 3 is a schematic view of an adhesive strip having a barrier strip made of a non-woven fabric; and
FIG. 4 is a pictorial view illustrating the application of said strip to the hairline.

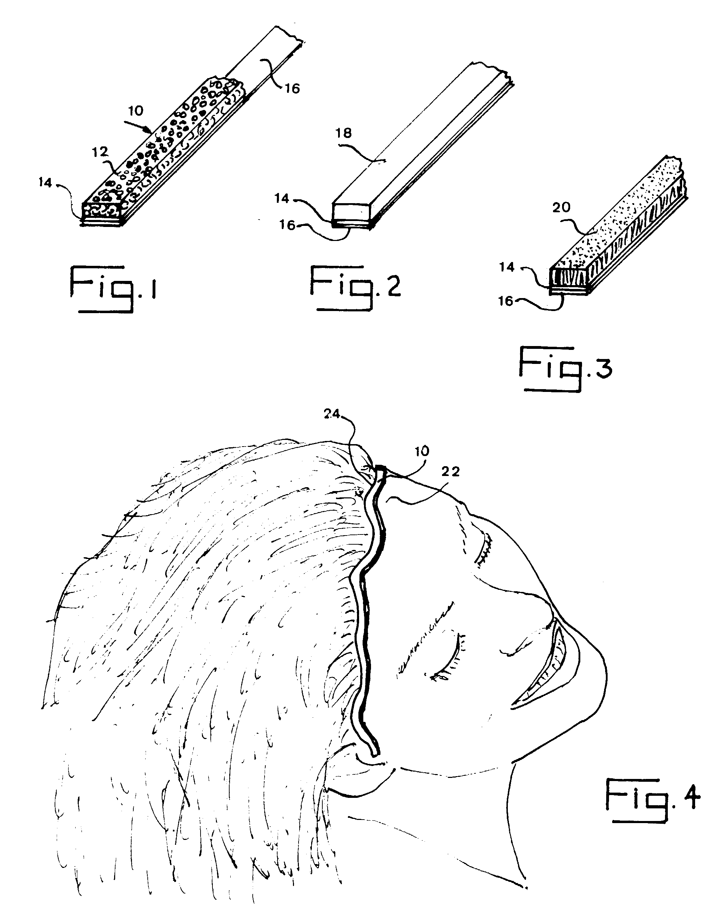

There is seen in FIG. 1 an adhesive strip 10 for preventing flow of hair treatment fluids to the skin of a user.

The major part of the strip comprises a barrier strip 12. Its thickness is between 1 to 8 mm, and its width between 4 and 10 mm.

The barrier strip 12 is made of an open cell material. A suitable cell size is 0.3 to 0.6 mm. Suitable materials include polyurethane, silicone, natural rubber, styrene butadiene rubber (SBR), polychloroprene, nitrile rubber, styrene butadiene styrene (SBS) rubber, styrene isoprene styrene (SIS) and polyvinyl chloride (PVC).

The lower face of the strip 12 is coated with an adhesive 14 which is self-sealing at a temperature of 30 - 38 degrees C. The adhesive 14 is preferably pressure sensitive. Advantageously the adhesive formulation is such that adhesive strength increases when its temperature rises from room temperature to body skin temperature, and adhesive strength decreases as a function of time in use as a result of contact with body secretions.

A suitable formulation for the adhesive 14 is achieved under the following conditions:

The adhesive contains between 20 to 85% tackifier, on a weight basis.

Suitable tackifiers are hydrocarbon resin, phenolic resin, polyterpene resin, terpene phenolic resin, wood rosin, hydrogenated wood rosin, and colophonium.

The adhesive base material comprises 10 - 35% polymer on a weight basis.

A preferred polymer base is a block co-polymer comprising at least one component compatible with body fat. Such component is suitably styrene butadiene styrene block polymer, styrene isoprene styrene block polymer, natural rubber, butyl rubber or styrene butadiene rubber.

The adhesive also contains 10 - 70% plasticizer on a weight basis which is compatible with body fat. Suitable plasticizers include paraffinic oils, naphthinic oils, aromatic oils, paraffin wax, dibutyl phthalate, dioctyl phthalate and polyolefinic oil. This oil may be polybutene or polyisobutene oil.

The exposed face of the adhesive 14 is protected by a removable release layer 16.

Referring now to FIG. 2, there is seen an embodiment wherein the barrier strip 18 is made of a non-woven fabric. Suitable materials for this embodiment include viscose, polyester, polypropylene, polyethylene, paper and crepe paper.

Shown in FIG. 3 is an embodiment wherein the barrier strip 20 is made of felt.

FIG. 4 shows the adhesive strip 10 in use, the release layer 16 having been removed. The strip 10 is applied to the skin 22 immediately adjacent to the hairline 24. In application a major portion of the adhesive coated bottom surface of the strip 10 is adapted to conform to the spatial curve defined by the hairline 24; such curve may be a compound curve. Due to the flexibility of the strip 10, as well as its moderate width, adhesion is effected without crimping or buckling, to form a continuous leak-proof barrier adjacent to the user's hairline 24 after attachment to the user's skin 22.

It will be evident to those skilled in the art that the invention is not limited to the details of the foregoing illustrative embodiments and that the present invention may be embodied in other specific forms without departing from the spirit or essential attributes thereof. The present embodiments are therefore to be considered in all respects as illustrative and not restrictive, the scope of the invention being indicated by the appended claims rather than by the foregoing description, and all changes which come within the meaning and range of equivalency of the claims are therefore intended to be embraced therein.

## Claims

1. An adhesive strip for preventing fluid communication between hair treatment agents and a user's skin, comprising:
a) a barrier strip having a thickness of about 1-8 mm and a width of about 4-10 mm, adapted to have a bottom surface thereof attached adjacent the hairline of a user;
b) a layer of heat-activated adhesive provided on the bottom surface of said barrier strip, said adhesive being self-sealing at body temperatures; and
c) a removable release layer attached to said adhesive,
a longitudinal edge of said adhesive coated bottom surface being adapted for contact with said hairline, and a major portion of the adhesive coated bottom surface of said strip being adapted to conform to a spatial curve defined by the contours of the hairline without crimping or buckling, said strip and adhesive forming a continuous leak-proof barrier adjacent the user's hairline after attachment to the user's skin.

2. An adhesive strip according to claim 1, wherein said barrier strip is made of an open cell material selected from the group consisting of polyurethane, silicone, natural rubber, styrene butadiene rubber (SBR), polychloroprene, nitrile rubber, styrene butadiene styrene (SBS) rubber, styrene isoprene styrene (SIS) and polyvinyl chloride (PVC).

3. An adhesive strip according to claim 2, wherein said open cell material has a pore size of about 0.3 to 0.6 mm.

4. An adhesive strip according to claim 1, wherein said barrier strip is made of a non-woven fabric.

5. An adhesive strip according to claim 4, wherein said non-woven fabric is selected from the group consisting of viscose, polyester, polypropylene, polyethylene, paper and crepe paper.

6. An adhesive strip according to claim 1, wherein said barrier strip is felt.

7. An adhesive strip according to claim 1, wherein said adhesive is a pressure-sensitive adhesive.

8. An adhesive strip according to claim 7, wherein said adhesive exhibits increased adhesive properties as temperature approaches body temperature and decreased adhesive properties as a function of time in use due to increased amounts of body secretions contacting said adhesive.

9. An adhesive strip according to claim 1, wherein said adhesive comprises about 20% to 85% wt/wt of a tackifier.

10. An adhesive strip according to claim 9, wherein said tackifier is selected from the group consisting of hydrocarbon resin, phenolic resin, polyterpene resin, terpene phenolic resin, wood rosin, hydrogenated wood rosin and colophonium.

11. An adhesive strip according to claim 1, wherein said adhesive comprises about 10%-35% wt/wt polymer base.

12. An adhesive strip according to claim 11, wherein said polymer base is a block co-polymer comprising at least one component which is compatible with body fat.

13. An adhesive strip according to claim 12, wherein said component is selected from the group consisting of styrene butadiene styrene block polymer, styrene isoprene styrene block polymer, natural rubber, butyl rubber and styrene butadiene rubber.

14. An adhesive strip according to claim 1, wherein said adhesive comprises about 5% to 70% wt/wt plasticizer which is compatible with body fat.

15. An adhesive strip according to claim 14, wherein said plasticizer is selected from the group consisting of parafinic oils, naphthinic oils, aromatic oils, parafin wax, dibutyl phthalate, dioctyl phthalate and polyolefinic oil.

16. An adhesive strip according to claim 15, wherein said polyolefinic oil is selected from the group consisting of polybutene and polyisobutene.

17. A method for preventing fluid communication between hair treatment agents and a user's skin, comprising:
i) providing an adhesive strip, said strip comprising:
a) a barrier strip having a thickness of about 1-8 mm and a width of about 4-10 mm, adapted to have a bottom surface thereof, attached adjacent the hairline of a user, said strip being made of a material characterized in that a longitudinal edge of said adhesive coated bottom surface is adapted for contact with said hairline, and a major portion of the adhesive coated bottom surface of said strip is adapted to conform to a spatial curve defined by the contours of the hairline without crimping or buckling;
b) an adhesive provided on the bottom surface of said strip; and
c) a removable release layer attached to said adhesive,
ii) removing said release layer; and
iii) applying said adhesive strip along the hairline of the user,
whereby said strip and adhesive forms a continuous leak-proof barrier adjacent thereto.
